Europäisches Patentamt

European Patent Office

Office européen des brevets

(11) Veröffentlichungsnummer: **0 265 741 B1**

(12) **EUROPÄISCHE PATENTSCHRIFT**

(45) Veröffentlichungstag der Patentschrift: **11.03.92**

(21) Anmeldenummer: **87114763.3**

(22) Anmeldetag: **09.10.87**

(51) Int. Cl.⁵: **A61B 17/22**

(54) **Flüssigkeitskreislauf für eine Vorrichtung zum Zertrümmern von Konkrementen im Körper eines Lebewesens.**

(30) Priorität: **24.10.86 DE 3636289**

(43) Veröffentlichungstag der Anmeldung:
**04.05.88 Patentblatt 88/18**

(45) Bekanntmachung des Hinweises auf die
Patenterteilung:
**11.03.92 Patentblatt 92/11**

(84) Benannte Vertragsstaaten:
**DE FR GB NL**

(56) Entgegenhaltungen:
**EP-A- 0 090 138**
**FR-A- 2 288 529**
**GB-A- 2 063 704**

(73) Patentinhaber: **SIEMENS AKTIENGESELL-
SCHAFT**
**Wittelsbacherplatz 2**
**W-8000 München 2(DE)**

(72) Erfinder: **Reitter, Josef**
**Elsterweg 3**
**W-8521 Möhrendorf(DE)**
Erfinder: **Tyukodi, Istvan**
**Sybelstrasse 3**
**W-8500 Nürnberg(DE)**
Erfinder: **Schindler, Reinhard**
**Adlerstrasse 3**
**W-8520 Erlangen(DE)**

## Beschreibung

Die Erfindung betrifft eine Vorrichtung zum Zertrümmern von Konkrementen im Körper eines Lebewesens mit wenigstens einem mit Flüssigkeit als Koppelmedium gefüllten Stoßwellengenerator und einem Flüssigkeitskreislauf, der einen Behälter und eine Umwälzpumpe aufweist, sowie Verfahren zum Betrieb der Vorrichtung.

In der DE-PS 32 10 919 ist eine derartige Vorrichtung beschrieben, bei der die zu untersuchende Person in einer mit Wasser gefüllten Wanne liegt. In einem Stoßwellengenerator mit einer Fokussierungskammer wird eine Stoßwelle z.B. durch Funkenentladung erzeugt, die auf das in dem Patienten befindliche Konkrement konzentriert wird und es zerkleinert. Die Fokussierungskammer ist durch eine feste Membran verschlossen und mit Flüssigkeit gefüllt. Über eine Umwälzpumpe wird die Flüssigkeit aus einem Vorrats-und Ausgleichsbehälter in die Fokussierungskammer gepumpt und im oberen Bereich wieder entnommen und zum Vorrats-und Ausgleichsbehälter geführt. Da aber der Vorrats-und Ausgleichsbehälter immer an dem Flüssigkeitskreislauf angeschlossen ist, ist es nicht gewährleistet, daß die Flüssigkeit, wie für die Lithotripsie erforderlich, ausreichend blasenfrei ist, so daß nur geringe Energieverluste oder Streuungen der Stoßwellen erfolgen.

Die Erfindung geht von der Aufgabe aus, eine Vorrichtung der eingangs genannten Art zu schaffen, bei der durch den Flüssigkeitskreislauf sichergestellt ist, daß die Flüssigkeit nur einen sehr geringen Anteil an Gasbläschen aufweist.

Die Aufgabe wird erfindungsgemäß dadurch gelöst, daß der Flüssigkeitskreislauf einen kleinen Kreislauf für den Normalbetrieb und einen großen Kreislauf für den Entgasungs- und Füllbetrieb aufweist, daß der kleine Kreislauf wenigstens einen Stoßwellengenerator, die Umwälzpumpe sowie einen Kühler und/ oder einen Blasenabscheider aufweist, daß in dem großen Kreislauf der Behälter eingeschlossen ist und daß in den Verbindungsleitungen zu den einzelnen Komponenten jeweils vor und nach der Umwälzpumpe Ventile zur Steuerung vorgesehen sind. Dadurch wird erreicht, daß der Entgasungsvorgang von zugeführter Flüssigkeit getrennt von dem kleinen Kreislauf erfolgt.

Ein leichtes Füllen und Entleeren wird ermöglicht, wenn durch weitere Ventile der Behälter mit einer Wasserzufuhr, einer Vakuumpumpe und einer Luftversorgung und der Flüssigkeitskreislauf mit einem Abfluß verbindbar ist. Es können auch störungsfrei zwei Stoßwellengeneratoren verwendet werden, wenn sie im kleinen Kreislauf parallelgeschaltet sind, wobei in den Zu- und Ableitungen jeweils ein steuerbares Ventil angebracht ist.

Der Füllvorgang des Behälters kann erfolgen, wenn zum Füllen des Flüssigkeitskreislaufes erst der Behälter durch Öffnen des Ventiles für die Wasserzufuhr gefüllt wird, wobei durch gleichzeitiges Öffnen des Ventiles der Behälter mit der nunmehr eingeschalteten Vakuumpumpe verbunden wird. Die zugeführte Flüssigkeit, beispielsweise Wasser, kann von Gasbläschen befreit werden, wenn zum Entgasen des Flüssigkeitskreislaufes der Behälter durch Öffnen des Ventiles mit der Vakuumpumpe verbunden wird, wobei gleichzeitig die in dem Behälter enthaltene Flüssigkeit umgewälzt wird. Der Füllvorgang des kleinen Kreislaufes mit blasenfreier Flüssigkeit kann erfolgen, wenn zum Auffüllen der Stoßwellengeneratoren zuerst das Ventil der Luftzufuhr kurzzeitig und anschließend die Ventile des großen Kreislaufes geöffnet werden und die Umwälzpumpe eingeschaltet wird, so daß der große Kreislauf eingeschaltet ist. Zum Betrieb des kleinen Kreislaufes müssen dessen Ventile geöffnet sein. Ein notwendiges Ablassen der Flüssigkeit, beispielsweise beim Auswechseln der Stoßwellengeneratoren, wird erreicht, wenn zum Entleeren des Flüssigkeitskreislaufes die Ventile des großen Kreislaufes sowie die Ventile für die Luftversorgung und den Abfluß geöffnet sind.

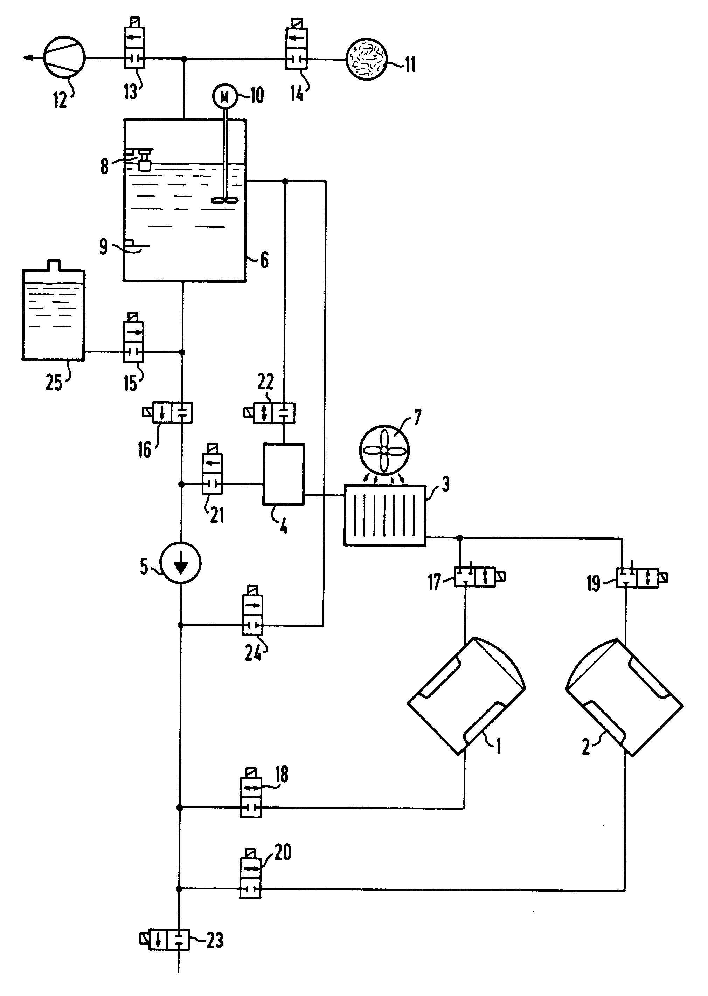

Die Erfindung ist nachfolgend anhand eines in der Zeichnung dargestellten Ausführungsbeispieles näher erläutert.

In der Figur sind zwei Stoßwellengeneratoren 1 und 2 schematisch dargestellt, wie sie beispielsweise aus der DE-OS 33 28 051 bekannt sind. Sie weisen eine Kammer auf, die mit einem Ankoppelmedium, beispielsweise mit Wasser, gefüllt ist. Damit keine Streuung der Stoßwellen erfolgt, muß diese Flüssigkeit möglichst blasenfrei sein.

Die Stoßwellengeneratoren 1 und 2 sind über Leitungen mit einem Kühler 3, einem Blasenabscheider 4 und einer Umwälzpumpe 5 zu einem kleinen Kreislauf zusammengeschlossen. Da die Stoßwellengeneratoren 1 und 2 auch einzeln betrieben werden können, sind in deren Zu- und Ableitungen jeweils elektrisch steuerbare Ventile 17 bis 20 vorgesehen, die einen Durchfluß in beiden Richtungen ermöglichen. Zwischen Blasenabscheider 4 und Umwälzpumpe 5 ist ein weiteres elektrisch steuerbares Ventil 21 vorgesehen, das in eine Richtung, der Pumprichtung der Umwälzpumpe 5, die Flüssigkeit durchläßt. Vom Boden eines Behälters 6 ist über ein elektrisch schaltbares Einwegventil 16 der Behälter 6 mit dem Ansaugstutzen der Umwälzpumpe 5 verbunden. Weiterhin ist der Behälter 6 seitlich in seinem mittleren Bereich über ein elektrisch steuerbares Einwegventil 24 mit der Umwälzpumpe 5 und über ein elektrisch steuerbares Zweiwegventil 22 mit dem oberen Bereich des Blasenabscheiders 4 verbunden. Im unteren Bereich ist der Behälter 6 über ein elektrisch steuerbares Einwegventil 15 mit einem Vorratsbehälter

2

25 als Wasserzufuhr verbunden. Nach oben hin ist der Behälter 6 über ein Einwegventil 13 mit einer Vakuumpumpe 12 und über ein Einwegventil 14 und einen Luftfilter 11 mit der Außenluft als Luftversorgung verbunden. Der Behälter 6 ist mit zwei Schwimmschaltern 8 und 9 für die Erfassung von unterschiedlichen Wasserhöhen versehen. Ein eventuell eingebautes, durch einen Motor 10 betriebenes Flügelrad bewirkt eine Umwälzung der Flüssigkeit im Behälter 6 und damit eine Beschleunigung der Entgasung.

Im nachfolgenden wird die Funktionsweise dieses Flüssigkeitskreislaufes für die einzelnen Betriebsphasen näher erläutert. Zum Füllen des gesamten Flüssigkeitskreislaufes werden manuell betätigt durch einen Schalter die Ventile 13 und 15 sowie die Vakuumpumpe 12 eingeschaltet, so daß von dem Vorratsbehälter 25 Wasser in den Behälter 6 gesaugt wird. Wird die erforderliche Wasserhöhe im Behälter 6 erreicht, schaltet der Schwimmschalter 8 das Ventil 15 für die Wasserzufuhr ab.

Nunmehr können eventuell vorhandene Gasbläschen während des Entgasungsvorganges aus dem im Behälter 6 enthaltenen Wasser entfernt werden. Dies erfolgt dadurch, daß entweder durch das vom Motor 10 betriebene Flügelrad oder durch die Umwälzpumpe 5, deren Zu- und Ableitungen durch die Ventile 16 und 24 freigegeben sind, eine langsame Umwälzung des Wassers erfolgt. Es kann aber auch eine weitere, nicht dargestellte Umwälzpumpe mit dem Boden und der Seite des Behälters 6 verbunden sein, die die Umwälzung des Wassers bewirkt. Die dabei austretenden Gasbläschen werden von der Vakuumpumpe 12 abgesaugt. Dieser Vorgang kann beispielsweise durch eine Zeitsteuerung erfolgen. Nach Ablauf der für die Entgasung erforderlichen Zeit werden die Umwälzpumpe 5 oder der Motor 10 sowie die Vakuumpumpe 12 und das Ventil 13 abgeschaltet.

Ist die in dem Behälter 6 enthaltene Flüssigkeit nach vorgegebener Zeit ausreichend entgast, so können nunmehr die Stoßwellengeneratoren 1 und 2 gleichzeitig oder nacheinander sowie der gesamte kleine Kreislauf gefüllt werden. Dazu wird zuerst das Ventil 14 für die Luftzufuhr angesteuert, so daß ein Druckausgleich in dem Behälter 6 erfolgen kann. Hierbei kann es vorteilhaft sein, daß ein geringer Unterdruck zum Ausgleich des Strömungswiderstandes in den Rückleitungen des kleinen Kreislaufes verbleibt. Anschließend werden die Ventile 16 bis 20 und 22 des großen Kreislaufes angesteuert, so daß das in dem Behälter 6 enthaltene Wasser in den kleinen Kreislauf fließ en kann. Zur Unterstützung wird die Umwälzpumpe 5 eingeschaltet. Dies kann so lange erfolgen, bis sich am Blasenabscheider 4 keine Blasen mehr zeigen. Nunmehr kann die Umwälzpumpe 5 abgeschaltet werden. Anschließend wird nach einer kurzen Zeit von beispielsweise einer Minute, in der sich das Wasser in dem kleinen Kreislauf beruhigt hat, der Behälter 6 durch Schließen der Ventile 16 und 22 abgeschaltet und das Ventil 21 geöffnet.

Nunmehr erfolgt eine Umwälzung des Wassers durch Einschaltung der Umwälzpumpe 5 nur noch im kleinen Kreislauf, während sich die Stoßwellengeneratoren 1 und 2 in einer Parkposition außerhalb der Betriebsstellung befinden. Eventuell noch vorhandene Blasen werden hierbei im Blasenabscheider 4 gesammelt.

Wurde durch das Füllen des kleinen Kreislaufes der untere Wasserstand im Behälter 6 unterschritten, so kann durch den zweiten Schwimmschalter 9 der Behälter 6 erneut gefüllt und das Wasser entgast werden, während der kleine Kreislauf inzwischen seinen Betrieb aufnehmen kann.

Für den Betrieb des Lithotripters wird wenigstens einer der Stoßwellengeneratoren 1 und 2 aus seiner Parkposition in die Betriebsstellung gebracht. Gleichzeitig wird durch Schließen der entsprechenden Ventilpaare 17 bis 20 jeweils der Kreislauf des nicht benutzten Stoßwellengenerators 1 oder 2 abgeschaltet, so daß die Flüssigkeit nur noch durch den eingeschalteten Stoßwellengenerator 1 oder 2 fließt. Durch diesen ständigen Flüssigkeitskreislauf während des Betriebes kann der Stoßwellengenerator 1 oder 2 gekühlt werden. Zur Unterstützung der Kühlwirkung kann dem Kühler 3 ein Ventilator 7 zur Luftzirkulation zugeordnet sein.

Nach Beendigung der Behandlung durch die Stoßwellengeneratoren 1 und 2 können diese wieder in ihre Parkposition gebracht werden, wobei wieder beide vom Wasser durchflossen werden.

Die Flüssigkeit in den Stoßwellengeneratoren 1 und 2 und dem kleinen Kreislauf kann von Zeit zu Zeit in vorgegebenen Intervallen automatisch aufbereitet werden. Vorher wird während des Betriebes des kleinen Kreislaufes gleichzeitig der Entgasungsvorgang im Behälter 6 durchgeführt. Hierzu wird wiederum das Ventil 13 geöffnet und die Vakuumpumpe 12 eingeschaltet und die Flüssigkeit in dem Behälter 6 umgewälzt. Dies kann durch das durch den Motor 10 betriebene Flügelrad oder durch die weitere, nicht dargestellte Umwälzpumpe erfolgen. Ist der Entgasungsvorgang abgeschlossen, wird wieder der oben beschriebene Druckausgleich herbeigeführt. Anschließend wird der große Kreislauf eingeschaltet, so daß die in dem Blasenabscheider 4 gesammelte Luft in den Behälter 6 gelangt. Gleichzeitig wird der kleine Kreislauf durch das in dem Behälter 6 entgaste Wasser nachgefüllt, so daß immer gewährleistet ist, daß die Koppelflüssigkeit in den Stoßwellengeneratoren 1 und 2 blasenfrei ist.

Sollen nun beispielsweise die Stoßwellengeneratoren 1 und 2 ausgewechselt werden, so muß das Wasser in dem Flüssigkeitskreislauf abgelassen werden. Zum Entleeren des Flüssigkeitskreislaufes werden die Ventile 14, 16 bis 23 geöffnet, so daß das in dem Flüssigkeitskreislauf enthaltene Wasser abfließen kann, während über die Luftversorgung Luft nachdringen kann, so daß ein eventuell entstehendes Vakuum verhindert wird.

**Patentansprüche**

1. Vorrichtung zum Zertrümmern von Konkrementen im Körper eines Lebewesens mit wenigstens einem mit Flüssigkeit als Koppelmedium gefüllten Stoßwellengenerator (1, 2) und einem Flüssigkeitskreislauf, der einen Behälter (6) und eine Umwälzpumpe (5) aufweist, **dadurch gekennzeichnet,** daß der Flüssigkeitskreislauf einen kleinen Kreislauf für den normalen Betrieb und einen großen Kreislauf für den Entgasungs- und Füllbetrieb aufweist, daß der kleine Kreislauf wenigestens einen Stoßwellengenerator (1, 2), die Umwälzpumpe (5) sowie einen Kühler (3) und/oder einen Blasenabscheider (4) aufweist, daß in den großen Kreislauf der Behälter (6) eingeschlossen ist, und daß in den Verbindungsleitungen zu den einzelnen Komponenten (1 bis 6) jeweils vor und nach der Umwälzpumpe (5) Ventile (16 bis 22, 24) zur Steuerung vorgesehen sind.

2. Vorrichtung nach Anspruch 1, **dadurch gekennkennzeichnet,** daß durch weitere Ventile (13 bis 15, 23) der Behälter (6) mit einer Wasserzufuhr (25), einer Vakuumpumpe (12) und einer Luftversorgung (11) und der Flüssigkeitskreislauf mit einem Abfluß verbindbar sind.

3. Vorrichtung nach Anspruch 1 oder 2, **dadurch gekennzeichnet,** daß zwei Stoßwellengeneratoren (1 und 2) parallelgeschaltet sind, wobei in den Zu- und Ableitungen jeweils ein steuerbares Ventil (17 bis 20) angebracht ist.

4. Verfahren zum Betrieb der Vorrichtung gemäß Patentanspruch 2 oder 3, wobei der Behälter (6) über ein Ventil (13) mit einer Vakuumentgasungseinrichtung (12) verbunden wird, **dadurch gekennzeichnet,** daß zum Füllen des Flüssigkeitskreislaufes erst der Behälter (6) durch Öffnen des Ventiles (15) für die wasserzufuhr (25) gefüllt wird und durch gleichzeitiges Öffnen eines anderen der weiteren Ventile (13) der Behälter (6) mit der nunmehr eingeschalteten Vakuumpumpe (12) verbunden wird.

5. Verfahren zum Betrieb der Vorrichtung gemäß Patentanspruch 2 oder 3, wobei der Behälter (6) über ein Ventil (13) mit einer Vakuumentgasungseinrichtung (12) verbunden wird, **dadurch gekennzeichnet,** daß zum Entgasen des Flüssigkeitskreislaufes der Behälter (6) durch Öffnen eines der weiteren Ventile (13) mit der Vakuumpumpe (12) verbunden wird, wobei gleichzeitig die in dem Behälter (6) enthaltene Flüssigkeit umgewälzt wird.

6. Verfahren zum Betrieb der Vorrichtung gemäß einem der Patentansprüche 2 bis 3, **dadurch gekennzeichnet,** daß zum Auffüllen der Stoßwellengeneratoren (1, 2) zuerst das Ventil (14) der Luftzufuhr (11) kurzzeitig und anschließend die Ventile (16 bis 22) des großen Kreislaufes geöffnet werden und die Umwälzpumpe (5) eingeschaltet wird, so daß der große Kreislauf eingeschaltet ist.

7. Verfahren zum Betrieb der Vorrichtung gemäß einem der Patentansprüche 1 bis 3, **dadurch gekennzeichnet,** daß zum Betrieb des kleinen Kreislaufes dessen Ventile (17 bis 21) geöffnet sind.

8. Verfahren zum Betrieb der Vorrichtung gemäß Patentanspruch 2 oder 3, **dadurch gekennzeichnet,** daß zum Entleeren des Flüssigkeitskreislaufes die Ventile (16 bis 22) des großen Kreislaufes sowie die Ventile (14, 23) für die Luftversorgung und den Abfluß geöffnet sind.

**Claims**

1. Apparatus for disintegrating concretions in the body of a living being with at least one shockwave generator (1, 2), filled with liquid as coupling medium, and a liquid circulation system which has a container (6) and a circulating pump (5), characterized in that the liquid circulation system has a small circulation system for normal operation and a large circulation system for the gas-extraction operation and filling operation, in that the small circulation system has at least one shockwave generator (1, 2), the circulating pump (5) as well as a cooler (3) and/or a bubble separator (4), in that the container (6) is enclosed in the large circulation system, and in that in the connection lines to the individual components (1 to 6) in each case before and after the circulating pump (5) there are provided valves (16 to 22, 24) for the control.

2. Apparatus according to claim 1, characterized in that through further valves (13 to 15, 23) the container (6) can be connected to a water supply (25), a vacuum pump (12) and an air supply (11) and the liquid circulation system can be connected to an outlet.

3. Apparatus according to claim 1 or 2, characterized in that two shockwave generators (1 and 2) are connected in parallel, whereby a controllable valve (17 to 20) is in each case fitted in the inlet and outlet lines.

4. Process for the operation of the apparatus in accordance with claim 2 or 3, whereby the container (6) is connected by means of a valve (13) to a vacuum gas-extraction device (12), characterized in that to fill the liquid circulation system first of all the container (6) is filled by opening the valve (15) for the water supply (25), and in that through simultaneous opening of an other one of the further valves (13) the container (6) is connected to the vacuum pump (12) which is now switched on.

5. Process for the operation of the apparatus in accordance with claim 2 or 3, whereby the container (6) is connected by means of a valve (13) to a vacuum gas-extraction device (12), characterized in that for the gas-extraction of the liquid circulation system the container (6) is connected to the vacuum pump (12) by opening one of the further valves (13), whereby at the same time the liquid contained in the container (6) is circulated.

6. Process for the operation of the apparatus in accordance with one of claims 2 to 3, characterized in that to fill the shockwave generators (1, 2) first of all the valve (14) of the air supply (11) is opened for a short time and subsequently the valves (16 to 22) of the large circulation system are opened and the circulating pump (5) is switched on so that the large circulation system is switched on.

7. Process for the operation of the apparatus in accordance with one of claims 1 to 3, characterized in that to operate the small circulation system its valves (17 to 21) are opened.

8. Process for the operation of the apparatus in accordance with claim 2 or 3, characterized in that to empty the liquid circulation system the valves (16 to 22) of the large circulation system as well as the valves (14, 23) for the air supply and the outlet are opened.

**Revendications**

1. Dispositif pour fragmenter des concrétions dans le corps d'un être vivant, comportant au moins un générateur d'ondes de choc (1,2) rempli par un liquide formant milieu de couplage, et un circuit de liquide, qui comporte un récipient (6) et une pompe de circulation (5), caractérisé par le fait que le circuit de liquide possède un petit circuit pour le fonctionnement normal et un grand circuit pour le fonctionnement de dégazage et de remplissage, que le petit circuit comporte au moins un générateur d'ondes de choc (1,2), la pompe de circulation (5) ainsi qu'un refroidisseur (3) et/ou un séparateur de bulles (4), que le récipient (6) est inséré dans le grand circuit, et que des soupapes (16 à 22, 24) utilisées pour la commande sont prévues dans les canalisations de raccordement des différents composants (1 à 6), respectivement en amont et en aval de la pompe de circulation (5).

2. Dispositif suivant la revendication 1, caractérisé par le fait que grâce à d'autres soupapes (13 à 15, 23), le récipient (6) peut être raccordé à une alimentation en eau (25), à une pompe à vide (12) et à une alimentation en air (11), et que le circuit de liquide peut être raccordé à une évacuation.

3. Dispositif suivant la revendication 1 ou 2, caractérisé par le fait que deux générateurs d'ondes de choc (1 et 2) sont branchés en parallèle, une soupape commandable (17 à 20) étant respectivement montée dans les canalisations d'amenée et d'évacuation.

4. Procédé pour faire fonctionner le dispositif suivant la revendication 2 ou 3, selon lequel le récipient (6) est raccordé par l'intermédiaire d'une soupape (13) à un dispositif de dégazage avec formation de vide (12), caractérisé par le fait que pour le remplissage du circuit de liquide, seul le récipient (6) est rempli moyennant l'ouverture de la soupape (15) pour l'amenée d'eau (25), et le récipient (6) est raccordé, au moyen de l'ouverture simultanée d'une autre des autres soupapes (13), à la pompe à vide (12) alors activée.

5. Procédé pour faire fonctionner le dispositif suivant la revendication 2 ou 3, selon lequel le récipient (6) est raccordé par une soupape (13) à un dispositif de dégazage avec formation de vide (12), caractérisé par le fait que pour dégazer le circuit du liquide, on raccorde le récipient (6) à la pompe à vide (12) en ouvrant l'une des autres soupapes (13), le liquide contenu dans le récipient (6) étant simultanément entraîné en circulation.

6. Procédé pour faire fonctionner le dispositif suivant l'une des revendications 2 à 3, caractérisé par le fait que pour le remplissage des générateurs d'ondes de choc (1,2), on ouvre tout d'abord la soupape (14) d'alimentation en air (11) pendant un bref intervalle de temps, puis les soupapes (16 à 22) du grand circuit et on branche la pompe de circulation (5) de sorte que le grand circuit est activé.

7. Procédé pour faire fonctionner le dispositif suivant l'une des revendications 1 à 3, caractérisé par le fait que pour faire fonctionner le petit circuit, on ouvre ses soupapes (17 à 21).

8. Procédé pour faire fonctionner le dispositif suivant la revendication 2 ou 3, caractérisé par le fait que pour vider le circuit de liquide, on ouvre les soupapes (16 à 22) du grand circuit ainsi que les soupapes (14 à 23) pour l'alimentation en air et l'évacuation.